# EUROPÄISCHE PATENTANMELDUNG

(11) **EP 4 091 566 A1**
(43) Veröffentlichungstag der Anmeldung: **23.11.2022**
(21) Anmeldenummer: 22174707.4
(22) Anmeldetag: 20.05.2022
(51) Int. Cl.: A61B 34/10

(54) **AUGMENTED REALITY-PREOPERATIVE PLANUNG**

(30) Priorität: 20.05.2021 DE 102021002652
(71) Anmelder: mediCAD Hectec GmbH, DE-84032 Altdorf (DE)
(72) Erfinder: ERDMANN, Eric, 84186 Vilsheim (DE)
(74) Vertreter: LS-MP von Puttkamer Berngruber Loth Spuhler

(57) **Zusammenfassung**

Verfahren, bei dem mittels eines Computers Maßnahmen zur Durchführung einer Operation geplant und Arbeitsschritte für die Operation an einem menschlichen oder einem tierischen Körper festgelegt werden, wobei die Operation den Einsatz wenigstens eines Implantats umfasst, der Computer ein Speicherwerk umfasst, in das Daten und Darstellungen in Form mehrdimensionaler Bilder eingespeichert werden, die Daten und Darstellungen den Körper eines Patienten betreffen, der der Operation unterzogen wird, und die Daten und Darstellungen eine Ausbildung des Implantats betreffen, das bei der Operation zum Einsatz kommt, wobei der Computer eine Bildassistenzeinrichtung umfasst, in die aus dem Speicherwerk, ausgewählte Daten und/oder Darstellungen eingespeist werden, wobei im Computer eine Simulation der geplanten Maßnahmen und/oder der Arbeitsschritte für die Operation durchgeführt und ausgewertet wird, die die Darstellung von Wechselwirkungen des Implantats mit dem Körper des Patienten umfasst, und die geplanten Maßnahmen und/oder die Arbeitsschritte für die Operation und/oder die Ausbildung des Implantats in Abhängigkeit des Auswertungsergebnisses der Simulation angepasst und/oder modifiziert werden, wobei mit Hilfe der Bildassistenzeinrichtung und auf Grundlage einer erweiterten Realität eine Visualisierung der Planung der Maßnahmen und/oder der Festlegung der Arbeitsschritte für die Operation und eine Visualisierung der Ausbildung des Implantats erfolgt.

## Beschreibung

Die Erfindung betrifft ein Verfahren sowie ein zugehöriges System zur Durchführung des Verfahrens, bei dem mittels eines Computers Maßnahmen zur Durchführung einer Operation geplant und/oder Arbeitsschritte für die Operation an einem menschlichen oder einem tierischen Körper festgelegt werden, nach den Merkmalen der Ansprüche 1 und 12.

Aus der DE 11 2011 100 810 T5 sind ein Verfahren und eine zugehörige Einrichtung zum Herstellen eines Implantats bekannt. Hierbei wird ein dreidimensionales Bild eines Gelenks des Patienten erstellt und es wird ein Implantat ausgewählt, das nicht individuell an das Gelenk des Patienten angepasst ist. Das Implantat wird nunmehr derart modifiziert, dass es patientenspezifisch ist. Hierbei werden mindestens sechs Winkel des Implantats modifiziert. Dabei werden anatomische und medizinische Informationen eines Patienten unter interaktiver Beteiligung eines Chirurgen integriert. Für einen bestimmten Patienten werden ein Implantat und chirurgische Instrumente im Wesentlichen aus drei Optionen ausgewählt und hergestellt: Sämtliche Implantatkomponenten, Ausrichtungsführungen und andere Einweginstrumente können in einem Paket enthalten sein, das dem Chirurgen für den jeweiligen Patienten zur Verfügung gestellt wird.

Im Hinblick auf viele, im Stand der Technik bekannte Verfahren und Einrichtungen zum Herstellen von Implantaten sowie zum Anpassen des jeweiligen Implantats an die Form und den Umfang des betroffenen Teils des Skeletts des Menschen oder des Tiers, wäre es wünschenswert, dem Operateur bei der Planung seiner operativen Maßnahmen eine verbesserte Einrichtung zur Visualisierung des Implantats und des Knochens, das mit dem Implantat in Verbindung gebracht werden soll, bereit zu stellen.

Eine verbesserte Visualisierung des Implantats und/oder des betroffenen Teils des Skeletts umfasst eine Verbindung aus mehrdimensionalen Bildern des Implantats und/oder des Knochens mit zugehörigen Daten und Informationen.

Es wäre darüber hinaus wünschenswert, dem Operateur für seine Planung Darstellungen, Maße und Alternativen zu dem Implantat optisch aufzubereiten und für einen schnellen Zugriff durch den Operateur bereit zu stellen.

Es wäre weiterhin wünschenswert, dem Operateur für seine präoperative Planung vorzugsweise wenigstens zweidimensionale Darstellungen des Implantats zugänglich zu machen. Auf diese Weise sollen dem Operateur alternative Implantatformen, Varianten und alternative Implantatmodelle von unterschiedlichen Herstellern zur Auswahl gegeben werden.

### Aufgabe der Erfindung

Die Erfindung stellt sich daher die Aufgabe, die mit dem Stand der Technik verbundenen Unzulänglichkeiten zu vermeiden.

### Lösung der Aufgabe

Die Aufgabe wird gelöst durch ein Verfahren und ein zugehöriges System, bei denen mittels eines Computers Maßnahmen zur Durchführung einer Operation geplant und/oder Arbeitsschritte für die Operation an einem menschlichen oder einem tierischen Körper festgelegt werden können.

Die Operation umfasst den Einsatz wenigstens eines Implantats.

Der Computer umfasst ein Speicherwerk, in das Daten und/oder Darstellungen in Form mehrdimensionaler Bilder eingespeichert werden können.

Die Daten und/oder die Darstellungen betreffen den Körper eines Patienten, der der Operation unterzogen werden soll.

Die Daten und/oder die Darstellungen betreffen weiter eine Ausbildung des Implantats, welches bei der Operation zum Einsatz kommen soll.

Der Computer umfasst eine Bildassistenzeinrichtung, in die aus dem Speicherwerk, ausgewählte Daten und/oder Darstellungen eingespeist werden.

Im Computer wird eine Simulation der geplanten Maßnahmen und/oder der Arbeitsschritte für die Operation durchgeführt und ausgewertet.

Die Simulation umfasst dabei die Darstellung von Wechselwirkungen des Implantats mit dem Körper des Patienten.

Die geplanten Maßnahmen und/oder die Arbeitsschritte werden für die Operation und/oder die Ausbildung des Implantats in Abhängigkeit eines Auswertungsergebnisses der Simulation angepasst und/oder modifiziert.

Mit Hilfe der Bildassistenzeinrichtung erfolgt auf Grundlage einer erweiterten Realität eine Visualisierung der Planung der Maßnahmen.

Auf Grundlage der erweiterten Realität erfolgt die Festlegung der Arbeitsschritte für die Operation.

Ebenfalls wird die Visualisierung der Ausbildung des Implantats mit Hilfe der Bildassistenzeinrichtung, auf Grundlage der erweiterten Realität ausgeführt.

Das vorliegende Verfahren und das dazugehörige System sind auf alle Bereiche des menschlichen und/oder tierischen Körpers anwendbar. Lediglich aus Vereinfachungsgründen wird im Folgenden nur noch von einem menschlichen Körper oder vereinfachend vom Patienten gesprochen.

Das Verfahren sowie das zugehörige System betreffen sämtliche Aspekte und Bestandteile des menschlichen Körpers. Dies sind beispielhaft, und in keiner Weise ausschließlich, der Kopf, die Haut, die Extremitäten sowie die Organe und das Skelett des Menschen.

Im Weiteren werden das Verfahren sowie das zugehörige System am Beispiel eines Knochens des Skeletts beschrieben.

### Computer

Die vorliegende Erfindung versteht unter dem Begriff "Computer" beispielhaft, und nicht ausschließlich, einen Personalcomputer (PC), ein Notebook, einen Tabletcomputer und/oder ein Smartphone und/oder ein Cloud-System einer Rechenanlage.

Der Computer kann wenigstens einen Daten-Stick, einen CD-Zugang und/oder einen DVD-Zugang umfassen.

Der Computer weist ein Speicherwerk auf.

Das Speicherwerk kann ein Datenarchiv oder ein mobiles und/oder feststehendes Speichermedium sein. In das Speicherwerk können Daten, Informationen, Bilder und Darstellungen abgespeichert werden.

Der Computer kann ein Rechner oder eine Rechenanlage sein. Es kann sich um eine Datenverarbeitungsanlage oder eine Anlage zur elektronischen Datenverarbeitung handeln.

Dem Computer können ein Eingabewerk und/oder ein Ausgabewerk zugeordnet sein.

Im Weiteren wird davon ausgegangen, dass es sich bei dem Computer um einen Digitalrechner handelt.

Der Computer umfasst vorzugsweise wenigstens eine Hardware-Komponente. Der Computer umfasst weiter wenigstens ein Softwareprogrammprodukt (Software).

Rein beispielhaft, aber in keiner Weise abschließend, umfasst der Computer ein Rechenwerk und/oder ein Steuerwerk.

Der Computer kann einen sogenannten Daten-Bus umfassen.

### Operation und Maßnahmen sowie Arbeitsschritte zur Durchführung der Operation

Gegenstand einer präoperativen Planung können operative Eingriffe sein. Beispielhaft, aber nicht ausschließlich, umfassen operative Eingriffe Knochenresektionen, das Einsetzen von Implantaten sowie die Auswahl von Implantaten nach bestimmten Parametern. Die Parameter können die Größe und der Typ des Implantats sein. Die Parameter können verschiedene geometrische Erfordernisse umfassen. Die geometrischen Erfordernisse können Bemessungen, wie z.B. die Höhe, die Breite, der Umfang, die Ausrichtung eines Skelett- und/oder eines Knochenteils sein. Die Parameter können einen Knochen, ein Weichteil, ein Gewebe und/oder ein Organen betreffen.

Bei einer chirurgischen Versorgung durch Einbringen eines Implantats werden zur präoperativen Planung 2D-Patientenbilder, in der Regel Röntgenbilder, gegenüber 3D-Patientenbildern bevorzugt. Bei der Erzeugung eines 3D-Patientenbildes wird der Patient mit einer geringeren Strahlendosis belastet, als bei einer Röntgenaufnahme.

Bei einem 2D-Patientenbild können eine Skalierung sowie die räumliche Tiefe in Blickrichtung der Aufnahme nicht, oder nur eingeschränkt, dargestellt werden. Dadurch ist es nur schwer zu beurteilen, ob ein Implantat richtig in oder an dem Knochen des Patienten anliegt. Es ist mit herkömmlichen Mitteln nur schwer zu beurteilen, ob ein Befestigungsmittel für das Implantat, z. B. eine Schraube, richtig in den Knochen eingreift oder ob sie über den Knochen hinausragt. Weiterhin bleibt oftmals unklar, ob die Schraube in den angrenzenden Knochen und/oder in das anliegende Gewebe und/oder in angrenzende Weichteile eindringen könnten.

Wenn ein Koordinatenbezug zwischen einem 2D-Patientenbild und einem 3D-Patientenbild hergestellt wird, können die jeweiligen Bearbeitungsschritte des Planers/Operateurs koordinatenbezogen in die andere Darstellungen übernommen werden. Hierdurch sind die Bearbeitungsschritte in sämtlichen ausgewählten Darstellungen planbar und prüfbar. Die Darstellungen umfassen insbesondere Belastungs- und Entlastungslagen des jeweiligen Körperabschnitts. Die Darstellungen umfassen zugehörige Dimensionen, vorzugsweise in einer Gegenüberstellung von Sollwerten und Istwerten.

Das erfindungsgemäße Verfahren betrifft auch Arbeiten und Planungsschritte, die der Planer/Operateur in der Arbeitsdarstellung, insbesondere im Zusammenhang mit einem ermittelten möglichen Implantat durchführt. Die Arbeiten und Planungsschritte umfassen vorzugsweise, aber nicht ausschließlich, die Positionierung eines möglichen Implantats anhand eines dargestellten Implantat-Modells. Die Positionierung kann an einem Wirbel oder an der Wirbelsäule die Veränderung der Form und/oder der Größe eines Implantat-Modells anzeigen. Die Verdrehung oder die Verschiebung des Implantat-Modells kann dargestellt werden. Ebenso kann die Veränderung des Anstellwinkels eines Implantat-Modells beispielsweise zu einem Wirbel gezeigt werden. Bemaßungen oder Messergebnisse können zeitgleich, und im Verhältnis zu den räumlichen Koordinaten, auf einem oder mehreren Bildschirmfenstern dargestellt werden.

Die präoperative Planung und/oder die Simulation eines operativen Eingriffs kann in einer oder in mehreren Darstellungen des Körperabschnitts des Patienten erfolgen, an dem der Eingriff durchgeführt wird. Die Darstellungen sind als Arbeitsdarstellungen ausgebildet, in denen wenigstens ein, im Verhältnis zum Körperabschnitt bewegbares Modell eines Implantats angeordnet werden kann. Auf die Simulation wird weiter unten gesondert eingegangen.

Die Erfindung versteht unter dem Begriff "Operation am menschlichen Körper" einen vorzugsweise instrumentellen, chirurgischen Eingriff, der am/oder im Körper eines Patienten ausgeführt wird.

Die Operation wird vorzugsweise zum Zweck der Therapie durchgeführt. Sie kann auch zum Zweck einer weiteren Diagnostik ausgeführt werden.

Die Operation kann vorzugsweise, aber nicht ausschließlich, ein gynäkologischer Eingriff sein. Es kann sich um eine Geburtshilfe handeln. Die Operation kann im Bereich der Urologie oder im Bereich der Hals-Nasen-Ohren-Heilkunde stattfinden.

Die Operation am menschlichen Körper, in der Folge vereinfachend Operation genannt, umfasst die präoperative Phase, die intraoperative Phase sowie die postoperative Phase.

### Implantat

Ein Implantat ist ein in den Körper einzusetzendes, insbesondere künstliches Material bzw. Teil, das permanent oder zumindest für einen längeren Zeitraum im Körper verbleibt.

Implantate werden vorzugsweise in medizinische, funktionelle oder plastische Implantaten unterschieden. Im Gegensatz zum Implantat wird z.B. eine Exoprothese außen am Körper angebracht. Im Weiteren wird beispielhaft, und nicht ausschließlich, von einem Implantat ausgegangen.

Medizinische Implantate können z.B. Herzschrittmacher, Stents, Gefäßprothesen, Kunstherzen oder Portkatheter sein. Es sind darüber hinaus Cochlea-Implantate sowie Retina-Implantate bekannt.

In der Zahnmedizin ersetzen Zahnimplantate mit Befestigungsankern die natürlichen Zähne.

Des Weiteren sind Implantate bekannt, die dazu dienen, ein Depot eines Arzneistoffs zu bilden. Nach Verbrauch des Arzneistoffs werden die Implantate ersetzt.

Eine weitere wichtige Gruppe sind Implantate, die z.B. beschädigte Gelenke ersetzen. Die Unfallchirurgie setzt Implantate zur operativen Behandlung von Knochenbrüchen ein. Bei Schädeldefekten nach einer Trepanation oder nach einer Resektion werden Implantate aus Titan zur individuellen Schädelrekonstruktion als Knochenersatz eingesetzt.

Funktionelle Implantate dienen z.B. der Überwachung von Tieren oder Menschen. Hierbei können RFID-Chips unter die Haut des Patienten eingepflanzt werden.

Plastische Implantate werden in der plastischen Chirurgie, z.B. als Ersatz für zerstörte Körperteile, oder auch zur Vergrößerung von vorhandenen Körperteilen verwendet.

Heutzutage werden die meisten operativen Eingriffe zur Einbringung eines Implantats durch ein rechnergestütztes Verfahren zum Abgleich von Patientendaten, insbesondere Patientenbildern, und möglichen Implantaten geplant, beispielsweise in Form von digitalen Bildern, die unter Verwendung einer Computer-Modellerstellungs-Software interaktiv erzeugt und geprüft werden können.

Im Weiteren ist das Implantat beispielsweise eine Schraube an einem Wirbelkörper der Wirbelsäule.

Für die Auswahl der Schraube steht eine Vielzahl von möglichen Parametern zur Verfügung. Auswahlkriterien sind z.B. der gewünschte Hersteller der Schraube, der Schraubentyp, die Ausführung der Schraube, das Schraubenmaterial sowie die Größe und die Länge der Schraube.

### Speicherwerk

Das Speicherwerk in diesem Sinne ist ein Datenträger. Das Speicherwerk kann ebenso ein integraler oder externer Bestandteil der Bildassistenzeinrichtung sein. Es kann der Speicher eines Personal Computers, eines Notebooks, eines Tabletcomputers, eines Smartphone, eines Speichersticks, eines optischen Datenträgers, eines Cloud-Systems oder eines sonstigen mobilen und/oder feststehenden beschreibbaren Speichermediums sein.

Die Patientenbilder und/oder weitere Patienteninformationen können beispielsweise in einem PACS-System (Bildarchivierungs- und Kommunikationssystem) oder einem anderen Datenspeicher enthalten sein. Sie können in elektronischer und/oder digitaler Form abgespeichert sein, wie beispielsweise auf einem magnetischen Speichermedium, einem optischen Speichermedium oder auf einem Halbleiterspeicher, einem sog. Cloud-System. Sie können darüber hinaus auf einer mobilen oder feststehenden Speichervorrichtung gespeichert werden.

Die Datenübertragung kann direkt von der Festplatte eines Computers erfolgen oder mittels eines anderen mobilen oder feststehenden Datenträgers einer Festplatte, einer CD, einer DVD, einer Flash-Speichervorrichtung (z. B. Speicherstift, Compact-Flash, sichere Digitalkarte), einem sog. Cloud-System.

Erfindungsgemäß können während des Planungsvorgangs neben den einzelnen Arbeitsschritten auch Kommentare und Anmerkungen zur Planung über die Bildassistenzeinrichtung gespeichert werden und verfügbar gemacht werden. Darüber hinaus können einzelne Arbeitsschritte, Kommentare oder Anzeigen in der Arbeitsdarstellung ein -und ausgeblendet werden.

Erfindungsgemäß können das endgültige Planungsergebnis eines möglichen zu planenden operativen Eingriffs und/oder Planungsschritte der Planung, also Teilergebnisse der Planung, jeweils als Datensätze kabellos, beispielsweise über Bluetooth oder sonstige mobile Übermittlungssysteme, oder kabelgebunden gespeichert werden, insbesondere in der Bildassistenzeinrichtung, aber auch in jedem anderen Speichermedium, wie beispielsweise einem externen Rechner, etc., oder in Archivsystemen.

Das Speicherwerk des Master-Computers und/oder des slave Computers kann ein PACS-System umfassen.

Das PACS-System ist ein Bildarchivierungs- und Kommunikationssystem.

Das PCS-System umfasst einen PACS-Server. Der PACS-Server kann an ein Kurzzeit- und ein Langzeitarchiv angeschlossen sein. Der PACS-Server sendet Daten und/oder Informationen an wenigstens einen Betrachtungs- und/oder Nachverarbeitungsrechner. Das PACS-System kommuniziert auch mit der Bildassistenzeinrichtung. Das PACS-System kann mit jeder weiteren Komponente des Computers kommunizieren.

### Bildassistenzeinrichtung

Die Bildassistenzeinrichtung im Sinne dieser Erfindung ist in der Lage, dem Planer/Operateur zugängliche Datenbestände zu visualisieren, zu skalieren und zu lesen, mindestens zwei mehrdimensionale Darstellungen zumindest eines Abschnitts des Patientenkörpers zu zeigen, von denen sich wenigstens zwei Darstellungen wenigstens in Bezug auf die Anzahl der Dimensionen unterscheiden.

Die Bildassistenzeinrichtung ist eine bevorzugt mit elektronischen Mitteln und zugehöriger Software arbeitende Einrichtung, die eine Schnittstelle hat und es ermöglicht, Bilddaten, anatomische Daten und geometrische Daten bei Erfordernis von Datenspeichern, mit bildgebenden Verfahren arbeitenden, nicht an die Einrichtung angebundenen Geräten oder Einrichtungen oder durch Handeingabe zu empfangen, zu verarbeiten und in einem softwaregebundenen Algorithmus so aufzubereiten, dass eine präoperative Planung und/oder eine Ausgabe von Planungsdaten an interoperativ benutzte Anzeige-, Hilfs-, Mess- oder bildgebende Aufnahmeeinrichtungen übermittelt, von diesen interoperativ abruft oder abruft und in Echtzeit verarbeitet und an die angeschlossenen Einrichtungen zurücksendet.

Bildgebendes Verfahren - ist jedes prä- oder interoperativ genutzte Verfahren, durch das Informationen über den Aufbau eines menschlichen oder tierischen Körpers oder Teilen davon oder dessen Eigenschaft ermöglicht und dessen aufgenommene Bilder zum Zweck präoperativer Planung und/oder interoperativer Kontrolle durch die Bildassistenzeinrichtung übernommen, ausgewertet, verarbeitet und über eine Schnittstelle an angeschlossene Anzeige-, Hilfs-, Mess- oder bildgebende Einrichtungen übermittelt werden kann.

Schnittstelle - ist eine Einrichtung, die unter Anwendung elektrischer, funktechnischer, lichttechnischer oder magnetischer Übertragungsverfahren auch unter Einbeziehung von programmtechnischen Algorithmen in der Lage ist, von der Bildassistenzeinrichtung generierte Daten an Anzeige-, Hilfs-, Mess-, bildgebende Aufnahme- oder Navigationseinrichtungen zu übermitteln und/oder Informationen von Anzeige-, Hilfs-, Mess- oder bildgebenden Aufnahmeeinrichtungen zu empfangen.

Das Navigationssystem - ist eine Einrichtung zur Unterstützung von Operationen. Sie dient bevorzugt der Einstellung erforderlicher geometrischer und räumlicher Verhältnisse. Sie kann die tatsächlichen geometrischen Verhältnisse erfassen und ist in der Lage, über die Schnittstelle der Bildassistenzeinrichtung sowohl Informationen von dieser zu empfangen als auch an diese zu liefern.

Steuerung - ist das über das präoperative Planungsverfahren hinausgehende Abrufen von durch die Bildassistenzeinrichtung generierten Bildinformationen durch Handeingabe, Gesten oder Informationen angeschlossener bildgebender Einrichtungen oder Informationen angeschlossener Einrichtungen zur Erfassung geometrischer Daten.

Weiterhin ist es ein Ziel der Erfindung, eine Skalierung der Informationen bei den Patientenbildern vornehmen zu können.

Erfindungsgemäß kann die präoperative Situation unter anderem durch eine automatische, halbautomatische oder manuelle Berechnung aller relevanten Winkel und sonstiger anatomischer oder operativer Parameter, etwa der Materialbeschaffenheit, der Größe etc. eines möglichen einzusetzenden Implantats, und der Patientendaten analysiert und dem Verfahren zu Grunde gelegt werden.

Das Verfahren zur Planung, Vorbereitung, Überwachung und Begleitung eines operativen Eingriffs wird an einer Bildassistenzeinrichtung durchgeführt, die dem Planer/Operateur eine archivierbare, speicherbare und reproduzierbare präoperative Planung ermöglicht und hierdurch seine Arbeit erleichtert.

Die Bildassistenzeinrichtung im Sinne dieser Erfindung ist in der Lage, dem Planer/Operateur zugängliche Datenbestände zu visualisieren, zu skalieren und zu lesen, mindestens zwei mehrdimensionale Darstellungen zumindest eines Abschnitts des Patientenkörpers zu zeigen, von denen sich wenigstens zwei Darstellungen wenigstens in Bezug auf die Anzahl der Dimensionen unterscheiden.

Die Bildassistenzeinrichtung erkennt die zugänglich gemachten Patientenbilder unter anderem in Bezug auf ihr Format z.B. als 3D-Bild und/oder 3D-Bilddaten bzw. als 2D-Bild und/oder 2D-Bilddaten.

Die Informationen können der Bildassistenzeinrichtung beispielsweise elektronisch, über das Internet, unter Verwendung geeigneter Übertragungsprotokolle und/oder auf anderem Wege, insbesondere für den Abruf, zum Einspielen bzw. Herunterladen und Abbilden, zugänglich gemacht werden.

Die Datenübertragung kann direkt von der Festplatte eines Computers erfolgen oder mittels eines anderen mobilen oder feststehenden Datenträgers einer Festplatte, einer CD, einer DVD, einer Flash-Speichervorrichtung (z. B. Speicherstift, Compact-Flash, sichere Digitalkarte), einem sog. Cloud-System.

Ferner sind verschlüsselte oder unverschlüsselte Übermittlungen per E-Mail oder über andere digitale Übermittlungen zu jedem geeigneten Typ von Computervorrichtung, Smartphone, PDA, Tabletcomputer oder zu anderen Vorrichtungen möglich, über die elektronische Informationen übermittelt werden können.

Es ist auch denkbar, der Bildassistenzeinrichtung aus einem 3D-Patientenbild gewonnene 2D-Schichten oder bereits vorhandene externe 2D-Schichten von 3D-Patientenbildern zugänglich zu machen und für die weitere Bearbeitung und/oder Planung des operativen Eingriffs sowie des Einsatzes eines orthopädischen Implantats zur Verfügung zu stellen. Gleiches gilt entsprechend für aus 2D-Patientenbildern gewonnene 3D-Bilder.

Die Bildassistenzeinrichtung kann zudem 2D-Schichten aus einem 3D-Patientenbild ableiten und errechnen und umgekehrt. Zur Darstellung der 2D-Schicht wird in den dreidimensionalen Körper, beispielsweise Knochen, Gewebe, Gelenke oder Weichteile, insbesondere den Wirbeln der Wirbelsäule, eine Ebene gelegt, die horizontal oder vertikal oder in einem Winkel zur Achse der Wirbelsäule verlaufen kann. Die Koordinaten der Ebene bestimmen die Koordinaten der 2D-Schicht und ermöglichen es, die Perspektive einer bestimmten zweidimensionalen Schicht des dreidimensionalen Körpers darzustellen. Die Berechnung der 2D-Daten kann automatisch erfolgen.

Ferner sind die 2D- und die 3D-Patientenbilder, die dem Verfahren zur Verfügung stehen, mit Hilfe der Bildassistenzeinrichtung in einer hochauflösenden MIP- bzw. einer MPR-Darstellung anzeigbar.

Ferner können erfindungsgemäß mit Hilfe der Bildassistenzeinrichtung 2D-Patientenbilder vor während oder nach deren Zugänglichmachung bearbeitet, insbesondere skaliert werden. Dabei werden automatisch, halb-automatisch oder manuell Abmessungen des abgebildeten Körperabschnitts des Patienten, insbesondere der Wirbelsäule, und/oder der Maßstab der Aufnahme mit Hilfe der Bildassistenzeinrichtung ermittelt, beispielsweise durch Verwenden eines bekannten Referenzobjekts, etwa einer Kugel aus einem nicht strahlendurchlässigen Material.

Der Skalierungsvorgang ist für jedes einzelne überspielte 2D-Patientenbild gesondert durchführbar und wiederholbar.

Die Bildassistenzeinrichtung ermöglicht es dem Planer/Operateur ferner, die zugänglich gemachten 3D-Patientenbilder in unterschiedlichen Ansichten, Ausschnitten und/oder Blickwinkeln zu segmentieren oder zu skalieren. Dies ermöglicht die Volumenerkennung und nicht nur die Erkennung einzelner Punkte der Patientenbilder. Die Segmentierung kann auch in einem Netz oder in einem Cloud-System erfolgen und gespeichert werden.

Die Segmentierung ermöglicht die Erkennung und medizinische/anatomische Bezeichnung und/oder Bezifferung einzelner Knochen, Wirbel, Gelenke, Weichteile, Gewebe etc. Die Segmentierung ermöglicht weiter die Erkennung und die medizinische/anatomische Bezeichnung und/oder die Bezifferung insbesondere der Wirbel der Wirbelsäule, anhand von Referenzmodellen und Soll-Werten. Sie ermöglicht den Vergleich mit medizinischen und anatomischen Ideal- und Normwerten der menschlichen oder tierischen Anatomie, um die durch die Operation angestrebte Patientenanatomie möglichst ideal planen zu können, die der Bildassistenzeinrichtung wie oben dargestellt zugänglich gemacht werden können. Zudem können mit Hilfe der Bildassistenzeinrichtung einzelne Knochen, Weichteile und Gewebeteile in den Darstellungen ein- oder ausgeblendet oder hervorgehoben werden.

Die Segmentierung mit Hilfe der Bildassistenzeinrichtung, also Erkennung und Bezeichnung insbesondere einzelner Knochen und/oder Skelettteile, Wirbel, Gelenke, Weichteile, Gewebe etc. kann insbesondere automatisch erfolgen und beispielsweise manuell überprüft werden.

Dies kann insbesondere bei Wirbeln, anhand von zugänglich gemachten Skizzen, insbesondere Wirbelskizzen, dargestellt, verdeutlicht und/oder überprüft werden.

Erfindungsgemäß können mit Hilfe der Bildassistenzeinrichtung an einem Bildschirm Bildschirmfenster mit unterschiedlichen Darstellungen, beispielsweise unterschiedliche Patientenbilder, Skizzen des operationsrelevanten Körperabschnitts, z.B. einer Wirbelsäule oder eines Wirbels, zeitgleich dargestellt werden.

Dabei ist es mit einem weiteren Aspekt der Erfindung möglich, mit Hilfe der Bildassistenzeinrichtung einen räumlichen Koordinatenbezug zwischen 2D-Patientenbildern und 3D-Patientenbildern herzustellen. Insbesondere kann eine Korrelation zwischen den vorzugsweise räumlichen Informationen von Daten und Informationen in 2D-Patientenbildern und 3D-Patientenbilder hergestellt werden und umgekehrt. Auf diese Weise sind Informationen aus einer Darstellung in eine andere Darstellung übertragbar, anzeigbar und bearbeitbar.

Das erfindungsgemäße Verfahren sieht des Weiteren die Möglichkeit vor, durch den Planer/Operateur mit Hilfe der Bildassistenzeinrichtung Patientenbilder, die in wenigstens einem Bildschirmfenster dargestellt werden, zu bearbeiten.

Insbesondere kann der Planer/Operateur beispielsweise durch Anklicken etwa mit einem Cursor, eine der Darstellungen in wenigstens einem Bildschirmfenster zur Bearbeitung in einer Arbeitsdarstellung aktivieren.

Unter Cursor in diesem Sinne ist jede Anzeige-, Eingabe-, Befehlsgebe- und/oder Bearbeitungsvorrichtung zu verstehen. Beispielsweise ist darunter eine Maus, ein Trackball, eine Tastatur, die Anzeige eines Touchscreen oder eines Grafiktabletts, insbesondere mit einem Zeiger, zu verstehen.

Dabei kann mindestens eine der mehrdimensionalen Darstellungen, insbesondere wenigstens eine 2D-Darstellung oder eine 3D-Darstellung, mit Hilfe der Bildassistenzeinrichtung als Arbeitsdarstellung aktiviert werden, wobei ein räumlicher Koordinatenbezug zwischen den einzelnen Darstellungen herstellbar ist.

Eine mit der 3D-Darstellung korrespondierende 2D-Darstellung kann ebenfalls mittels des Cursors bearbeitet werden. Dabei können 2D-Darstellungen und/oder 2D-Schichten z.B. mit Hilfe des Scrollrades einer Maus oder eines ähnlichen rechnerverbundenen Zeigemechanismus durchlaufen werden. Die jeweilige Arbeitsposition wird zeitgleich und in den entsprechenden räumlichen Koordinaten auf der jeweiligen Darstellung in jedes andere Bildschirmfenster übertragen. Das erfindungsgemäße Verfahren ermöglicht so eine genaue Orientierung und Übertragung von Koordinaten sowohl in 2D-Darstellungen als auch in korrespondierenden 3D-Darstellungen eines dargestellten Abschnitts des Patientenkörpers. Der Cursor verdeutlicht, welchen Bereich einer 3D-Darstellung und/oder einer 2D-Darstellung der Planer/Operateur zum jeweiligen Zeitpunkt markiert und/oder beplant und/oder bearbeitet.

Derartige Bearbeitungsmaßnahmen etwa in 3D-Patientenbildern können vorzugsweise automatisch in ihren Auswirkungen auch in 2D-Patientenbildern, vorzugsweise unter Herstellung eines Koordinatenbezuges, wiedergegeben werden. Gleiches gilt umgekehrt, wenn die Bearbeitungsmaßnahmen in 2D-Patientenbildern vorgenommen werden.

Externe Geräte können Bildassistenzeinrichtungen, Anzeige-, Hilfs-, Messgeräte oder vorzugsweise ein mobiles oder ein stationäres (Operations-) Navigationssystem sein. Das Navigationssystem dient insbesondere zur Steuerung und Überwachung eines operativen Eingriffs, insbesondere zur Bestimmung der örtlichen Position und/oder zur Bestimmung der Koordinaten des Eingriffs und der Einleitung und der Durchführung von operativen Maßnahmen. Die Planungsdaten können beispielsweise kabellos oder kabelgebunden über geeignete Protokolle übermittelt werden.

Die Schnittstelle kann ferner so konfiguriert sein, dass sie in der Lage ist, entfernt angeordnete Anzeigegeräte mit Informationen zu versorgen. Beispielsweise kann dies ein in einem Operationssaal angeordneter Bildschirm sein.

Die Schnittstelle kann so ausgelegt sein, dass sie nicht nur Daten von externen Quellen übernehmen kann oder nur Steuerdaten an eine Anzeigeeinrichtung liefern kann. Bei entsprechender Ausrüstung der Schnittstelle ist es möglich, permanent Daten von externen Quellen zu laden, diese mit gespeicherten Daten zu vergleichen und über eine Anzeigeeinrichtung dem Operateur zur Verfügung zu stellen. Mögliche Daten sind dabei neben den oben beschriebenen Bilddaten auch durch externe Messeinrichtungen ermittelte geometrische Daten, beispielsweise Daten über Lage, Ausrichtung oder Fortschritt eines operativen Eingriffs.

Dabei ist es unter anderem möglich, sog. elektronische Zäune, beispielsweise zur Begrenzung des Operationsbereichs und/oder zur Markierung von bestimmten Bereichen im Körper des Patienten, zur genauen Positionierung eines Implantats in der mehrdimensionalen, vorzugsweise dreidimensionalen Planung, festzulegen und zur Ein- und/oder Ausgrenzung dieser Bereiche sowie zur örtlichen Koordination des Eingriffs und/oder des Implantats vorzunehmen und die zugrunde liegenden Koordinaten an ein mobiles oder stationäres Operations-Navigationssystem kabellos oder kabelgebunden zu überspielen.

Es ist weiterhin möglich, die bei der präoperativen Planung ermittelten Daten in ein 3D-Modell des Implantats zu übersetzen und diese Daten über die Schnittstelle an mit dieser verbundene Maschinen zu senden. Dies können beispielsweise spanabhebende Werkzeugmaschinen sein, die entweder ein neues Implantat herstellen oder vorhandene Implantate individuell nachbearbeiten. Ebenso ist es möglich, einen 3D-Drucker anzusteuern, der entweder ein angepasstes Implantat herstellt oder ein nicht als Implantat geeignetes Muster erzeugt, das abhängig vom Bedarf Studienzwecken während der präoperativen Planung dient, als Modell für Lehrzwecke geeignet ist oder auch als Modell für ein durch Urformen herzustellendes Implantat dienen kann.

Eine weitere Möglichkeit besteht darin, dass die Ergebnisse der präoperativen Planung über die Schnittstelle einem Navigationssystem übermittelt werden, wobei entweder direkt Steuerdaten übermittelt werden oder das Navigationssystem aus erhaltenen Planungs- oder Bilddaten selbst Steuerdaten gewinnt.

Es ist ebenso möglich, dass die Bildassistenzeinrichtung über seine Schnittstelle nicht nur Daten an angeschlossene Geräte liefert. Es kann ebenso über die Schnittstelle eine Rückübertragung von Daten der externen Geräte an die Bildassistenzeinrichtung durchgeführt werden. Damit wird es möglich, durch ständigen Vergleich der Soll-, Planungs- und Ist-Daten während der Operation eintretende Veränderungen festzustellen, um faktisch ein Monitoring der durchgeführten Operation zu ermöglichen.

Neben der automatischen Erfassung von Daten und dem automatischen Abgleich ist des Weiteren eine zusätzliche visuelle Überwachung der Vorgänge im zu operierenden Körperteil beziehungsweise im Körper zu erkennen, die der Operateur möglicherweise nicht erkennen kann.

Kann die Bildassistenzeinrichtung oder die Schnittstelle ein Monitoring ausführen oder ermöglichen, wird dadurch gewährleistet, über die Schnittstelle auch Daten oder Befehle an angeschlossene externe Geräte zu übermitteln. Insbesondere bei der Verwendung eines Navigationssystems besteht so die Möglichkeit, auf Abweichungen von geplanten Maßnahmen und dem Planungsergebnis aufmerksam zu machen und somit Unregelmäßigkeiten bei der Durchführung der Operation zu vermeiden. Die Art der gegebenen Hinweise kann von den Fähigkeiten des Navigationsgerätes abhängen. Es kann beispielsweise eine optische und/oder akustische Information ausgegeben werden.

Die Bildassistenzeinrichtung kann über die Schnittstelle Bilddaten an einen Monitor im Operationssaal ausgeben. In Fällen, in denen der Operateur Details der präoperativen Planung oder aktuelle Veränderungen nachvollziehen muss, kann über die Schnittstelle eine Gestensteuerung an das Bildassistenzeinrichtung angekoppelt werden. So besteht die Möglichkeit, trotz vorhandener hygienischer Bedingungen im Operationssaal zeitnah nachzuprüfen, ob die Operation mit ihren Ergebnissen den Vorgaben des Planungsergebnisses entspricht.

Dieses Planungsergebnis kann über die Schnittstelle der Bildassistenzeinrichtung mit Patientenbildern, insbesondere 2D- und/oder 3D-Patientenbildern korreliert werden. Dabei wird eine oder mehrere Röntgen-, CT-, MRT- oder andere in einem bildgebenden Verfahren hergestellte Aufnahme des Patienten in der Lage auf dem Operationstisch erstellt.

Zusätzlich besteht die Möglichkeit, die Aufnahme mit bekannten Verfahren zu kalibrieren. Mit Hilfe der Schnittstelle der Bildassistenzeinrichtung wird eine Korrelation der Koordinaten der mehreren Patientenbilder hergestellt, so dass das Patientenbild des Patienten auf dem Operationstisch in Koordination, Ausrichtung und Darstellung mit der Darstellung des Planungsergebnisses in der Bildassistenzeinrichtung, insbesondere der Navigationsansicht übereinstimmt.

Über die Schnittstelle der Bildassistenzeinrichtung und des Navigationssystems ist es zudem möglich, das Planungsergebnis und/oder die Teile des Planungsergebnisses und/oder das mindestens auf dem Operationstisch von dem Patienten erstellte Patientenbild einer bildgebenden Vorrichtung, beispielsweise einem Röntgengerät, einem MRT-Gerät oder einem CT-Gerät zur Verfügung zu stellen. Zudem ist es über die Schnittstelle der Bildassistenzeinrichtung und des Navigationssystems möglich, der Bildassistenzeinrichtung und dem Navigationssystems Daten und Informationen zur Verfügung zu stellen, zu speichern, anzuzeigen und darzustellen.

Erfindungsgemäß kann das Planungsergebnis und/oder Teile des Planungsergebnisses und/oder Patientenbilder, die von den Patienten auf dem OP-Tisch angefertigt wurden, an eine bildüberwachende Vorrichtung kabelgebunden oder kabellos übermittelt werden. Die bildüberwachende Vorrichtung ermittelt nach bekannten Verfahren virtuell Lage und Position des Patienten sowie die Lage, die Position und den Fortschritt von einzelnen Schritten der geplanten Operation.

Auf Grund der Kalibrierung sind die tatsächlichen Abmessungen und Dimensionen des Patienten mittels der Bildassistenzeinrichtung gespeichert und können dem Navigationssystem über eine Schnittstelle übermittelt werden.

Instrumente oder Implantate sind beispielsweise aus der verfahrensgemäßen Planung ebenfalls mittels der Bildassistenzvorrichtung gespeichert oder werden mit Hilfe der Bildassistenzeinrichtung zugänglich gemacht, beispielsweise unter Zugriff auf bekannte gespeicherte Informationen oder durch bildgebende und kalibrierte Aufnahmen der Instrumente oder Implantate.

Die Bildassistenzeinrichtung und das Navigationssystem ermöglichen eine automatische Korrelation der Position, Ausdehnung und der Lage des Patienten mit der Position, Ausrichtung und Lage von Implantaten oder Instrumenten.

Zusätzlich können die Instrumente oder Implantate mit wenigstens einem Ortungsmittel verbunden sein, welche die relative Position der Instrumente oder Implantate und Veränderungen in Position, Ausdehnung und Lage der Instrumente oder Apparate an die Bildassistenzeinrichtung und das Navigationssystem übermitteln können.

Mit Hilfe der Bildassistenzeinrichtung und des Navigationssystem können alle Werkzeuge, Implantate und die Lage des Patienten relativ zueinander so dargestellt werden, wie sie auch tatsächlich im Raum liegen.

Insbesondere werden die Führungslinien der geplanten Eingriffe und der geplanten Implantate sowie elektronische Zäune mit Hilfe des Navigationssystem oder der Bildassistenzeinrichtung angezeigt. Dem Operateur ist es somit möglich, in einem Echt-Zeit-Vergleich die einzelnen Operationsschritte an die präoperativ geplanten Operationsschritte anzupassen. Der Operateur kann erfindungsgemäß insbesondere Lage, Position, Winkel, Tiefe, Positionierung und Durchführung der präoperativ geplanten Schritte an das Planungsergebnis anpassen. Der Operateur kann hierdurch verhindern, beispielsweise zu tief und/oder in einem falschen Winkel oder an der falschen Stelle Operationsschritte vorzunehmen.

Erfindungsgemäß wird zudem eine Vorrichtung zur Ausführung eines operativen Eingriffs nach dem erfindungsgemäß erlangten Planungsergebnis eines operativen Eingriffs und/oder Teilergebnisse der Planung zur Verfügung gestellt und zur Ausführung eines erfindungsgemäß geplanten operativen Eingriffs verwendet.

Die in die Bildassistenzeinrichtung eingespeisten Daten und/oder Darstellungen werden vorzugsweise bearbeitet und/oder verarbeitet.

Die in die Bildassistenzeinrichtung eingespeisten Daten und/oder Darstellungen umfassen Implantat-Typen und Abmessungen von Implantaten unterschiedlicher Herstellung. Zusätzlich sind in die Bildassistenzeinrichtung von verschiedenen Herstellern angefertigte auf dem Markt erhältliche Implantat-Varianten umfasst.

### Darstellungen und/oder Daten

Patientenbilder sind unter anderem Röntgenaufnahmen, bei denen es sich um 2D-Aufnahmen bzw. -Bilder handelt, sowie volumenbasierte Aufnahmen, wie beispielsweise CT oder MRT, bei denen es sich um 3D-Aufnahmen bzw. -Bilder handelt.

Volumenbasierte Aufnahmen, also 3D-Aufnahmen bzw. Bilder, bestehen in der Regel aus mehreren Schichten von 2D-Bildern (sog. 2D-Schichten). Diese können in einzelne 2D-Bildschichten unterteilt werden.

Volumenbasierte Bilder werden im Gegensatz zu 2D-Aufnahmen bzw. Bildern bereits bei der Aufnahme skaliert. Dies bedeutet, dass Größe des aufgenommenen Gegenstands und Maßstab der Aufnahme bekannt sind. Die jeweiligen Datenangaben zur Skalierung des Bildes werden gemeinsam mit der jeweiligen volumenbasierten Aufnahme als Datensatz gespeichert.

Demgegenüber müssen 2D-Bilder in der Regel von dem Planer/Operateur zur Planung eines operativen Eingriffs im Vorfeld der Planung skaliert werden, um Maßstab und mögliche Verzerrungen in der Tiefe des dargestellten Abschnitts eines Patientenkörpers festlegen zu können.

Planungsdaten können hier in Form der 2D-Bilddaten vorliegen bzw. aus diesen z. B. durch Bildbetrachtung oder -vermessung gewonnen werden.

Bekannte rechnergestützte Systeme zur präoperativen Planung können dem Planer/Operateur zusätzlich zur Darstellung von Patientenbildern und von möglichen Implantaten auch eine Auswahl eines bestimmten Implantats und dazugehöriger Instrumente, die bei dem chirurgischen Eingriff zu verwenden sind, vorschlagen. Gleiches gilt für Gattungen derartiger Implantate oder Instrumente.

Bei der bekannten Verwendung nur von 2D-Patientenbildern oder nur von 3D-Patientenbildern zur Planung eines operativen Eingriffs besteht jedoch der Nachteil, dass beispielsweise Verschiebungen von Gelenken, Belastungen, Entlastungen oder Verformungen von Knochen und Gelenken, insbesondere pathologische Verformungen der Wirbelsäule wie beispielsweise Kyphose oder Lordose, oder unterschiedliche Belastungsprofile nicht in Aufnahmesystemen abbildbar und deshalb nicht in die präoperative Planung einbeziehbar sind.

So werden beispielsweise Volumenpatientenbilder beim liegenden Patienten aufgenommen; hier erfolgt also eine Aufnahme des Patienten häufig in einem entlasteten Zustand.

2D-Patientenbilder, z.B. Röntgenaufnahmen, der Wirbelsäule, werden hingegen häufig bei stehendem Patienten aufgenommen, weil die Aufnahmesysteme diese Lage einfacher darstellen können und der betreffende Körperteil in seinem tatsächlichen Belastungszustand wiedergegeben werden kann.

Insbesondere bei der Planung von Eingriffen an der menschlichen oder tierischen Wirbelsäule, aber auch an deren Gelenken oder Skelettteilen, ist es jedoch notwendig, unterschiedliche Aufnahmesituationen und Lagen des Patienten, insbesondere stehende und liegende Situationen, beispielsweise zur Bestimmung der sagittalen Balance, einbeziehen zu können, zumal der operative Eingriff am liegenden Patienten vorgenommen wird.

Erforderlich ist es deshalb, die sich aus 2D- und 3D-Patientenbildern ergebenden Informationen - insbesondere was den Belastungs- und Entlastungszustand von Skelettteilen, Knochen, Gelenken, Wirbeln, Gewebe, Weichteilen anbelangt - dem Planer des operativen Eingriffs/Operateur auf einfache Weise zur Verfügung zu stellen, so dass er sie vorzugsweise gleichzeitig betrachten und weiterverarbeiten kann.

Patientenbilder sind beispielsweise durch bildgebende Verfahren entstandene zwei- oder dreidimensionale Aufnahmen, die durch bekannte Verfahren zu erhalten sind. Bevorzugt handelt es sich um 2D-Aufnahmen aus der Anwendung von Röntgenverfahren beziehungsweise um 3D Aufnahmen aus der Anwendung von CT oder MRT. Soweit im Folgenden von 2D- und/oder 3D-Patientenbildern die Rede ist, wird hierunter neben der eigentlichen Abbildung auch der zugrunde liegende Datensatz verstanden.

Soweit nicht anders ausgeführt, werden die Begriffe Information und Daten im Folgenden synonym verwendet.

Die Patientenbilder und Patienteninformationen können beispielsweise zu verschiedenen Zeitpunkten entstanden sein, 2D- und/oder 3D-Bild-Datensätze des Patienten und/oder, weitere Daten umfassen, insbesondere zu Körpergröße, Gewicht, Alter des Patienten, und etwa Daten aus vorangegangenen Eingriffen, Daten aus vorherigen Untersuchungen und/oder solche Daten umfassen, die in unmittelbaren Zusammenhang mit einer Operation erstellt wurden.

Darüber hinaus können der Bildassistenzeinrichtung zur Durchführung des erfindungsgemäßen Verfahrens weitere Informationen, wie z.B. Patientenbilder, Bemaßungsdaten, Funktionsdaten und/oder sonstige Referenzdaten, Soll-Werte, Standard-Werte und/oder allgemeine patientenunspezifische Daten und/oder Informationen zu Implantaten, wie beispielsweise 2D- und 3D-Modelle von Implantaten, Herstellerinformationen, Artikelnummer, Bemaßung, Material, Verwendungsanweisungen etc., der Implantate, und/oder Skizzen sowie Norm-, Ideal- oder Sollwerte der menschlichen oder tierische Anatomie zugänglich gemacht werden.

Patientenspezifische und allgemeine Informationen können insbesondere mit den mehreren mehrdimensionalen Patientenbildern dargestellt, abgebildet und/oder abgespeichert werden.

Die Darstellungen und die dazugehörigen Daten umfassen vorzugsweise mehrdimensionale Bilder.

Die mehrdimensionalen Bilder sind vorzugsweise 2D-Darstellungen oder 3D-Darstellungen.

### Simulation der geplanten Maßnahmen und/oder der Arbeitsschritte

Die vorliegende Erfindung versteht unter dem Begriff "Simulation" eine Vorgehensweise, nach der der Operateur die Maßnahmen zur Durchführung der Operation am menschlichen Körper und/oder die Arbeitsschritte, die für die Operation erforderlich sind, analysiert.

Im Rahmen der Simulation der Maßnahmen und/oder Arbeitsschritte für die Operation führt der Operateur einen Soll-Ist-Vergleich durch.

In diesem Soll-Ist-Vergleich stellt er vorzugsweise mit Hilfe der Bildassistenzeinrichtung eines Computers die vor Beginn der Operation geplanten Maßnahmen zum Vergleich neben die tatsächlich vor Beginn der Operation durchgeführten Maßnahmen.

Zur Simulation der Maßnahmen und/oder Arbeitsschritte, die für die Operation erforderlich sind, stellt der Operateur mit Hilfe der Bildassistenzeinrichtung eines Computers die geplanten Arbeitsschritte, die zur Durchführung der Operation erforderlich sind neben die tatsächlich während der Operation ausgeführten Arbeitsschritte.

Vorzugsweise mit Hilfe der Bildassistenzeinrichtung des Computers werden die vom Operateur im Rahmen der Simulation geplanten Maßnahmen und/oder die geplanten Arbeitsschritte für die Operation am menschlichen Körper durchgeführt und ausgewertet.

Im Rahmen der Simulation werden Wechselwirkungen des Implantats mit den Bereichen des Skeletts und/oder mit den betroffenen Knochen ermittelt und ausgewertet.

Aufgrund der Ergebnisse der Auswertung der Simulation werden die geplanten Maßnahmen und/oder zur Operation erforderlichen Arbeitsschritte und/oder die Ausbildung des Implantats in Abhängigkeit des Auswertungsergebnisses der Simulation angepasst und/oder modifiziert.

Die Wechselwirkung bezeichnet dabei die verschiedenen Möglichkeiten, nach denen die physikalischen Objekte "Knochen" und "Implantat" einander beeinflussen.

Die gegenseitige Einflussnahme des Implantats auf den Knochen und/oder umgekehrt kann Wechselwirkungen umfassen, die die Gravitation betreffen oder einen Elektromagnetismus.

Es kann eine schwache oder eine starke Wechselwirkung zwischen dem Implantat und dem jeweilig betroffenen Knochen vorliegen.

Informationen, Daten und/oder Werte in Bezug auf den Knochen und/oder in Bezug auf das Implantat können zu Anpassungen und/oder Modifikationen führen. Die Anpassungen und/oder Modifikationen können Maßnahmen und/oder Arbeitsschritte betreffen, die für die Operation am menschlichen Körper erforderlich sind.

Die Anpassung und die Modifikation kann überdies auch das Implantat betreffen, welches im Rahmen der Operation in den Körper des Menschen eingesetzt werden soll.

Die modifizierten oder angepassten Maßnahmen und/oder die angepassten oder modifizierten Arbeitsschritte für die Operation sowie die Daten und/oder die Darstellung des angepassten oder modifizierten Implantats werden zur Abspeicherung in das Speicherwerk des Computers übermittelt.

Während des Planungsprozesses kann simuliert werden, zu der betroffenen Anatomie gehöriges Weichgewebe zu modifizieren oder zu entfernen oder zu reparieren, um zum Beispiel die Ausrichtung eines Gelenks wiederherzustellen oder gerissenes oder krankes Gewebe zu entfernen oder Bänder zu schneiden oder zu reparieren oder natürliche oder künstliche Bändertransplantate vorzusehen. Informationen über Weichgewebe können wahlweise als zusätzliche Auslegungsparameter für die Auswahl eines geeigneten Implantats verwendet werden.

Der Vorteil des erfindungsgemäßen Verfahrens liegt darin, durch die mögliche gleichzeitige Darstellung und Bearbeitung mehrerer mehrdimensionaler Patientenbilder Maßnahmen der Planung, insbesondere der Simulation, eines operativen Eingriffs an unterschiedliche Anatomiemodalitäten des Patienten in unterschiedlichen Aufnahmen, insbesondere 2D-Patientenbildern und 3D-Patientenbildern, anzupassen. Ein weiterer Vorteil liegt darin, dass das Verfahren ungünstige biomechanische Ergebnisse vermeiden und durch eine optimierte Implantationsgeometrie ersetzen kann.

Die jeweilige Arbeitsdarstellung dient insbesondere der Bearbeitung und /oder Simulation eines operativen Eingriffs durch den Planer/Operateur.

Die Bearbeitungs- und/oder Simulationsschritte des Planers/Operateurs in einer Arbeitsdarstellung können insbesondere rechnergesteuert etwa mit Hilfe eines Cursors oder anderen Eingabemethoden erfolgen.

Durch Markieren, beispielsweise Anklicken eines Knochens, Gelenks, Weichteils oder Gewebes in der Arbeitsdarstellung, insbesondere eines oder mehrerer Wirbel der Wirbelsäule, kann der Planer/Operateur in der wenigstens einen Arbeitsdarstellung insbesondere an einem Bereich der Wirbelsäule, in einem 2D-Patientenbild und/oder in einem 3D-Patientenbild Planungsschritte durchführen und einzelne Operationsschritte simulieren.

Mit Hilfe der Bildassistenzeinrichtung werden die zugänglich gemachten Daten, insbesondere Bemaßungen, Messergebnisse etc. innerhalb eines Vergleiches der individuellen Patientendaten mit Skizzen oder Soll-, Ideal- oder Normwerten bewertet und auf Abweichungen zwischen Soll- und Ist-Werten untersucht, um ein optimales post-operatives Ergebnis für diesen Patienten zu erzielen.

Mit Hilfe der Bildassistenzeinrichtung, insbesondere mit Hilfe der wenigstens einen Arbeitsdarstellung, können fachspezifische Bemaßungen, wie etwa Länge, Tiefe, Umfang, Höhe und Breite und/oder Dichte, beispielsweise eines Knochens und/oder Skelettteils, eines Gewebes, eines Weichteils oder Organs, in Form von Referenzpunkten dargestellt werden anhand derer Referenzpunkte an Implantat-Modellen, die in einer Datenbank gespeichert sein können, abgeglichen werden und so automatisch oder manuell passende Implantate, beispielsweise Cages (Implantat-Käfige), Bauteile, künstliche Gelenke, Schrauben, Platten, Fixateure, Implantatkomponenten, Ausrichtungsführungen, Einweg-Instrumente, Befestigungsmittel, Pfanne und Schaft für ein Hüftimplantat etc., für den jeweiligen Patienten automatisch ausgewählt und vorgeschlagen werden.

In einem vorzugsweise automatischen Auswahlverfahren zur Planung eines operativen Eingriffs werden anhand des Abgleichs von bekannten und/oder ermittelten Referenzpunkten zwischen Implantat-Informationen und den Patientenbildern wenigstens ein am besten passendes Implantat ermittelt, vorgeschlagen und automatisch mit Hilfe der Bildassistenzeinrichtung in eine der Darstellungen, z.B. in das 3D-Patientenbild, vorzugsweise in die wenigstens eine Arbeitsdarstellung eingeblendet.

Insbesondere ist es dem Planer/Operateur in der wenigstens einen Arbeitsdarstellung möglich, bei der Planung und/oder Simulation eines operativen Eingriffs an der Wirbelsäule durch Markieren, insbesondere Anklicken mit dem Cursor, wenigstens ein Teil beispielsweise eines Wirbels auf einem Patientenbild zu entfernen. Ferner ist es möglich, durch Markieren, insbesondere Anklicken, etwa mit einem Cursor andere Teile am Wirbel anzuordnen oder z.B. ein Implantat am Wirbel zu befestigen. Je nach der benötigten Perspektive kann in einer Arbeitsdarstellung eine 3D-Darstellung mit Hilfe etwa eines Cursors durch den Planer/Operateur gedreht, geschwenkt und/oder geneigt werden.

Es ist insbesondere auch vorstellbar, Schnitte durch einen beliebigen dargestellten Körperteil, etwa durch Wirbel, und weitere mögliche Schritte der zu planenden und durchzuführenden Operation zu simulieren, um nachvollziehen zu können, welche Auswirkung eine bestimmte Anordnung eines Implantats oder ein bestimmter Operationsschritt in Bezug auf den inneren Bereich des Körperteils hat.

Erfindungsgemäß ist es insbesondere zur präoperativen Planung von Wirbelsäulenoperationen beispielsweise möglich, auf Basis der Patientenbilder die sagittale Balance rechnergestützt zu vermessen. Hierzu werden auf wenigstens einer der Darstellungen in den Bildschirmfenstern von dem Planer/Operateur Bestimmungspunkte mit Hilfe des Cursors markiert. Dies sind insbesondere der ventrale Endpunkt der Sakrumbasis, der ventral-craniale Endpunkt der Sakrumbasis und/oder die beiden Femurkopf-Mittelpunkte. Die Messergebnisse können rechnergestützt darüber hinaus einem Soll-/Ist-Vergleich mit Soll-, Ideal- und Normwerten der menschlichen oder tierischen Anatomie unterworfen werden und in einer Ergebnisliste aufgeführt und bewertet werden. Auf diese Weise kann insbesondere ein mögliches einzusetzendes Implantat nach bestimmten Parametern ausgewählt und positioniert werden.

Für die weitere Bearbeitung an der jeweiligen Position auf einem 3D-bzw. 2D-Patientenbild wird in der wenigstens einen Arbeitsdarstellung mit Hilfe des Cursors durch den Planer/Operateur ein Implantat-Modell in die 3D- bzw. 2D-Darstellung eingefügt.

Dabei können mit dem erfindungsgemäßen Verfahren zunächst die gewünschten Parameter der Schraube mit Hilfe der Bildassistenzeinrichtung festgelegt werden. In einem nächsten Schritt erfolgt die Auswahl des operationsrelevanten Knochens, insbesondere eines Wirbelkörpers, z.B. in einer Skizze und/oder auf einem Patientenbild in einer der Darstellungen in den Bildschirmfenstern. In wenigstens einer Darstellung in einem weiteren Bildschirmfenster sind ausgewählte Ausschnitte darstellbar.

Das an Hand vorgegebener Parameter ausgewählte Implantat-Modell, beispielsweise eine Schraube, kann rechnergestützt in der Arbeitsdarstellung in einem mehrdimensionalen Patientenbild vorplatziert werden. Zeitgleich wird das Implantat-Modell in der ausgewählten Form in weiteren Darstellungen in den Bildschirmfenstern an den entsprechenden räumlichen Koordinaten des jeweils in den Darstellungen abgebildeten Patientenkörpers angezeigt. Die Positionierung der Schraube an einem Wirbel in den übrigen Bildschirmfenstern entspricht dabei der Anordnung und Ausrichtung der Schraube an einem Wirbel in der Arbeitsdarstellung.

Dabei kann in der Arbeitsdarstellung die Positionierung eines Implantat-Modells, beispielsweise einer Schraube, ohne Abänderung der Neigung, der Richtung sowie des Winkels etwa mit Hilfe des Cursors, beispielsweise durch Ziehen mit dem Cursor auf oder entlang der X-, Y- und/oder der Z-Achse, verschoben und/oder auf oder entlang von Kreisen, die jeweils die Drehachse bzw. die mögliche Drehrichtung wiedergeben, gedreht werden.

Es besteht ferner die Möglichkeit, für ein Implantat-Modell, beispielsweise eine Schraube, in der Arbeitsdarstellung eine Bohrung in einem Knochen, beispielsweise einem Wirbel, zu simulieren, wobei die Tiefe der Bohrung durch den Planer/Operateur verlängert oder verkürzt werden kann.

Es ist ferner möglich, in der Arbeitsdarstellung mit Hilfe des Cursors die Veränderung des Neigungswinkels, des Drehwinkels sowie des Schwenkwinkels des Implantat-Modells, beispielsweise einer Schraube, in einem Knochen, beispielsweise einem Wirbel, zu simulieren. Hierbei wird die Simulation auf andere benachbarte Knochen, insbesondere Wirbel übertragen,unter Beibehaltung der simulierten Form und Güte sowie unter Beibehaltung der simulierten Ausrichtung gegenüber dem dargestellten Knochen. Vorzugsweise erfolgt die Simulation anhand von Wirbeln der Wirbelsäule im Vergleich zu den umliegenden Knochen und Weichteilen, beispielsweise den Wirbeln der Wirbelsäule.

Auf die dargestellte Weise können unterschiedliche Implantate und deren räumliche Anordnung am Patientenbild simuliert und im Hinblick auf ihre Auswirkung und Verträglichkeit gegenüber benachbarten Knochen, Gewebe, Weichteilen, Organen, etc. geplant werden. Der Operateur hat somit die Möglichkeit, für jeden Patienten das optimale Implantat sowie die optimale Ausrichtung des Implantats zu planen und/oder zu simulieren.

Die Erfindung erweist sich auch deshalb als vorteilhaft, da die Ergebnisse des operativen Eingriffs, insbesondere des Einsatzes wenigstens eines orthopädischen Implantats durch die zeitgleiche Darstellung in wenigstens zwei Bildschirmfenstern und aus unterschiedlichen Blickwinkeln bzw. Perspektiven der Darstellungen weiterer Bildschirmfenster kontrolliert und gegebenenfalls korrigiert werden können. Dies erfolgt unabhängig davon, ob die simulierte Handlung in einer 3D- oder einer 2D-Arbeitsdarstellung erfolgt ist oder ob wenigstens ein weiteres Bildschirmfenster neben der Arbeitsdarstellung geöffnet ist.

Das Verfahren ermöglicht insbesondere, zur Planung von Wirbelsäulenoperationen die Berücksichtigung von Nash-Moe-Rotation, von innerem Pedikel-Abstand, von Spinalkanal-Weitenindex, von Lordose, von Kyphose, von atlantodentale Distanzen, des Durchmesser des Wirbelkanals, von Wirbellinien, der Mc Gregor's-Linie, von Spondylolisthese, der Bandscheibenhöhe, der Bandscheibenwinkel, der Instabilität nach van Akkerveeken, des sakralen Winkels, von Funktionsanalysen, von Stabilitätskriterien bei der Spondiloretrolysthese, der Anordnung von Grund- oder Deckplatten, der Anordnung eines Lots zum Wirbelkörper-Schwerpunkt, der zervikale Schwerelinie, von Rissergrad, von Skoliose nach Cobb oder nach Ferguson, von Pelvic Tilt PT und/oder von Pelvic Incidence PI, von Pelvic Angulation PA, von Pelvic Lordosis Angle, von Pelisacral Angle PSA und/oder von Sacral Slope, von C7 Plumb Line, von Pelvic Thickness CS, von Pelvic Thickness SPT , etc., mit denen einzelne Schritte eines operativen Eingriffs an der Wirbelsäule in einer Arbeitsdarstellung simuliert werden können. Insbesondere aus der Anterior/Posterior- und der Sagittal-Ansicht der Wirbelsäule können zudem beispielsweise Apex-Winkel und tatsächlicher Winkel bestimmt und dem Verfahren zu Grunde gelegt werden.

Ferner kann durch das erfindungsgemäße Verfahren eine biometrische Analyse erstellt und ein Vorschlag für die optimierte Lage von Gelenkmittelpunkten erstellt werden. Körpergröße, Gewicht und die biomechanische Ausgangsanalyse des Patienten können automatisch berücksichtigt werden. Bei der Durchführung einer biometrischen Analyse kann mit dem erfindungsgemäßen Verfahren beispielsweise der Drehpunkt für eine optimierte Gelenkgeometrie simuliert werden, wobei die Belastungssituation eines gesunden Gelenkes zu Grunde gelegt werden kann. Dergleichen Bemaßungen und Messergebnisse können rechnerisch ermittelt und angezeigt werden.

Zur Planung der Osteotomie kann die Bildassistenzeinrichtung Teilbilder aus einzelnen Darstellungen und/oder Patientenbildern zu einer einzigen Darstellung, beispielsweise zur Simulation der Verbindung von Knochenenteilen, zusammenfügen. Darüber hinaus kann der Operateur mit Hilfe der Bildassistenzeinrichtung einfache Bildbearbeitungen für eine Rekonstruktion von Skelettelementen durchführen.

Ein weiterer Vorteil der Erfindung besteht darin, dass zur Planung der Osteotomie Knochensegmente freigestellt, verschoben und erforderliche Osteosynthese-Implantate platziert werden können. Fehlstellungswinkel können z.B. nach Dror Paley berechnet werden.

Zudem können zur Osteosynthese geeignete und/oder erforderliche Komponenten wie Nägel, Platten, Schrauben etc. ausgewählt werden.

Mit Hilfe des Verfahrens kann des Weiteren ein operativer Eingriff an Schultern, Ellenbogen, Händen und Fingern geplant und die Auswahl und die Position von Implantaten optimal vorbereitet werden.

Das erfindungsgemäße Verfahren kann auch eine Verbesserung der Krafteinleitung und der Wiederherstellung des physiologischen Muskelspiels an Hand von biomechanisch ermittelten Normbereichen, nach denen die Implantate verankert werden, ermöglichen.

Gleiches gilt auch für die Planung eines operativen Eingriffs am menschlichen und/oder tierischen Fuß, am Sprunggelenk oder am Zeh, beispielsweise der nach außen gerichteten Schiefstellung der Großzehe im Grundgelenk (Hallux Valgus).

Das erfindungsgemäße Verfahren ermöglicht es dem Planer/Operateur darüber hinaus, Achsfehlstellungen zu ermitteln und automatisch oder manuell zu korrigieren.

Prä- und auch postoperative Messergebnisse können verfahrensgemäß in der wenigstens einen Arbeitsdarstellung angezeigt werden.

Erfindungsgemäß können das endgültige Planungsergebnis eines möglichen zu planenden operativen Eingriffs und/oder Teilergebnisse der Planung über eine Schnittstelle an ein nicht zur Bildassistenzeinrichtung gehörendes Gerät übermittelt werden. Es ist möglich, Daten und/oder Befehle an externe Geräte zu übermitteln, beispielsweise an eine weitere Bildassistenzeinrichtung in einem Operationssaal.

Auf diese Weise können das endgültige Planungsergebnis eines möglichen zu planenden operativen Eingriffs und/oder Teilergebnisse der Planung im Operationssaal dargestellt werden.

Nach einem vorteilhaften Aspekt der Erfindung wird das Planungsergebnis und/oder Teilergebnisse der Planung an eine Bildassistenzeinrichtung im Operationssaal oder ein Navigationssystem (47) übermittelt. Dort kann die Arbeitsdarstellung als Navigationsansicht aufgerufen werden, so dass das Planungsergebnis und/oder Teilergebnisse der Planung mit Führungslinien angezeigt werden.

Dieses Planungsergebnis kann über die Schnittstelle der Bildassistenzeinrichtung mit Patientenbildern, insbesondere 2D- und/oder 3D-Patientenbildern korreliert werden. Dabei wird eine oder mehrere Röntgen-, CT-, MRT- oder andere in einem bildgebenden Verfahren hergestellte Aufnahme des Patienten in der Lage auf dem Operationstisch erstellt.

### Visualisierung

Unter dem Begriff "Visualisierung" versteht die Erfindung, das Sichtbarmachen oder graphisch Darstellen der Maßnahmen zur Durchführung einer Operation am menschlicher Körper.

Es ist ebenfalls gemeint, die Sichtbarmachung und Veranschaulichung der für die Operation erforderlichen Arbeitsschritte.

Gleichfalls ist mit dem Begriff "Visualisierung" die Sichtbarmachung des Implantats in den zur Verfügung stehenden Varianten und Ausprägungsformen gemeint, auf die der Operateur zurückgreifen kann.

Mit Hilfe der Visualisierung kann der Operateur die Simulation der zur Durchführung der Operation geplanten Arbeitsschritte und/oder zur Durchführung der Operation geplanten Maßnahmen mit Hilfe der Bildassistenzeinrichtung optisch und/oder graphisch darstellen.

Mit Hilfe der Visualisierung kann der Operateur auch die Wechselwirkungen darstellen, die das Implantat auf das Skelett und/oder den jeweiligen Knochen des Skeletts des Patienten ausübt.

Die Visualisierung der geplanten und/oder durchgeführten Maßnahmen sowie die geplanten und/oder durchgeführten Arbeitsschritte bei der Operation werden mit Hilfe eines Visualisierungsmittels der Bildassistenzeinrichtung veranschaulicht.

Gleiches gilt auch für das und/oder die zur Verfügung stehenden Implantate.

Mit Hilfe der Visualisierung werden auch Anpassungen und Modifikationen veranschaulicht, die in Abhängigkeit von dem Ergebnis der Simulation an den Maßnahmen und/oder an den Arbeitsschritten zur Operation durchgeführt und vorgenommen werden.

Gleiches gilt auch für die Anpassungen und die Modifikationen, die aufgrund des Ergebnisses der Simulation am Implantat vorgenommen werden.

Das Visualisierungsmittel kann mit Hilfe einer Gestensteuerung und/oder einer Sprachsteuerung und/oder manuell gesteuert werden.

Unter dem Begriff "Visualisierungsmittel" versteht die Erfindung eine Einrichtung, mit der vorzugsweise abstrakte Daten, Texte und/oder Zusammenhänge in einer graphischen und/oder visuell erfassbaren Form dargestellt werden können.

Zur Darstellung der Daten, Inhalte, Texte und/oder Zusammenhänge in der graphischen bzw. visuell erfassbaren Form ist das Visualisierungsmittel vorgesehen.

Das Visualisierungsmittel ist als eine optische Einrichtung ausgebildet. Das Visualisierungsmittel kann ein Smartphone sein. Es kann auch als Brille ausgebildet sein.

Das Visualisierungsmittel kann als optische Einrichtung ausgebildet sein und an einem Körperteil der Person angeordnet sein, die an der Visite teilnimmt. Vorzugsweise ist die optische Einrichtung am Kopf des Arztes angeordnet.

Die optische Einrichtung ist schwenkbar ausgebildet. Sie kann aus einer Ruhestellung im Bereich des Kopfes in eine Arbeitsstellung verschwenkt werden, in der die Einrichtung wenigstens vor einem Auge des Arztes positioniert ist.

Die Einrichtung ist in einem Abstand vor dem wenigstens einen Auge des Arztes angeordnet, sodass dem Arzt die abstrakten Daten, Texte und sonstigen Zusammenhänge graphisch und/oder visuell erfassbar dargestellt werden können.

Das Visualisierungsmittel umfasst ein Speichermedium zur Abspeicherung der Daten, Werte und Informationen.

Die Daten werden dem Visualisierungsmittel vorzugsweise drahtlos und/oder über eine WLAN-Verbindung aus dem Informationssystem des Krankenhauses übermittelt.

Das Visualisierungsmittel umfasst weiter eine Rechnereinheit, mit der die Daten, Werte und Informationen bearbeitet werden können.

Es versteht sich von selbst, dass die Werte, Informationen und Daten in der Rechnereinheit auch verarbeitet werden können, um sie im Visualisierungsmittel optisch darstellen zu können.

Auf die Rechnereinheit des Visualisierungsmittels wird weiter unten im Detail eingegangen.

Zur Herstellung einer Kommunikation zwischen dem Visualisierungsmittel und dem Informationssystem des Krankenhauses umfasst das Visualisierungsmittel wenigstens eine Schnittstelle.

Über die Schnittstelle kann auch eine Kommunikation zwischen dem Visualisierungsmittel und einem Gerät aufgebaut werden, das vorzugsweise im Krankenzimmer steht. Das Gerät dient zur Überwachung des Patienten oder zur Durchführung weiterer Therapiemaßnahmen. Es versteht sich von selbst, dass das Gerät auch für andere Aufgaben und Funktionen eingesetzt werden kann.

Das Visualisierungsmittel wird wenigstens einer Person zugeordnet, die an der Visite teilnimmt.

Es versteht sich von selbst, dass das Visualisierungsmittel auch mit Hilfe einer APP funktionieren kann, die vorzugsweise, aber nicht ausschließlich, in das Handy oder ein Tablet oder ein sonstiges elektronisches Gerät eingespielt wurde.

Von der Bildassistenzeinrichtung kann wenigstens eine Skizze erzeugt werden. Die Skizze ist eine Darstellung des operationsrelevanten Abschnitts eines Patientenkörpers, wie sie etwa in allgemeinen biologischen oder anatomischen Darstellungen verwendet wird. Skizzen in diesem Sinne dienen insbesondere als Hilfsmittel zur besseren Visualisierung, Lokalisierung etc. des dargestellten Abschnitts des Körpers in 2D- und/oder 3D-Patientenbildern. Skizzen, insbesondere Wirbelsäulenskizzen, können insbesondere den operationsrelevanten Abschnitt des Patientenkörpers, insbesondere Skelettteil, Knochen, Gelenke, Gewebe, Weichteile etc. insbesondere in seitlicher Darstellung, insbesondere als 3D-Modell, anzeigen. Diese Skizzen können unter anderem Soll-, Ideal- und Normwerte der menschlichen oder tierischen Anatomie beinhalten und mit Hilfe der Bildassistenzvorrichtung einen Vergleich der Werte des individuellen Patienten mit Norm-, Ideal- oder Sollwerten herstellen.

Zunächst ist es ein Ziel der vorliegenden Erfindung, die den 2D-Patientenbildern oder den 3D-Patientenbildern jeweils zu Grunde liegenden Informationen so zusammenzuführen oder zusammen berücksichtigen zu können, dass sie für die Visualisierung und/oder Bearbeitung des jeweils anderen Patientenbildes zur Verfügung stehen; insbesondere soll es möglich sein, die sich aus dem unbelasteten Zustand des Körpers in einem 3D-Patientenbild ergebenden Informationen für die Visualisierung und/oder Bearbeitung des 2D-Patientenbildes übertragen zu können, also die Informationen und Darstellung des 2D-Patientenbildes durch die Informationen und Darstellung des 3D-Patientenbildes zu ergänzen, zu ändern oder sonst wie zu modifizieren, bzw. umgekehrt.

Erfindungsgemäß kann der Planer/Operateur der Bildassistenzeinrichtung gleichzeitig oder nacheinander mehrere auswählbare mehrdimensionale Patientenbilder, insbesondere in Form von 2D-Patientenbildern und 3D-Patientenbildern, zugänglich machen, abrufen, einspielen bzw. herunterladen und zeigen.

Die Bildassistenzeinrichtung steuert den Aufbau des Bildschirms in der Form, dass der Bildschirm entweder ein einzelnes Bildschirmfenster aufweist oder in mehrere Bildschirmfenster unterteilbar ist. Der Bildschirm kann horizontal, vertikal, diagonal oder in jeder anderen Form, insbesondere zeitgleich in wenigstens zwei gleich oder unterschiedlich große Bildschirmfenster unterteilt werden.

In dem wenigstens einen Bildschirmfenster kann jeweils eine Darstellung gezeigt werden. Die Darstellungen können insbesondere Patientenbilder, Skizzen, insbesondere Wirbelsäulenskizzen, Befundungsmonitore, sonstige Abbildungen oder Daten, beispielsweise zu Implantaten umfassen. Darüber hinaus können Patientenbilder eines größeren Körperabschnitts aber auch einzelne Knochen, Gelenke, Weichteile und/oder Gewebe, insbesondere einzelne Wirbel, in einem Bildschirmfenster dargestellt werden.

Die Darstellungen wenigstens zweier Bildschirmfenster können zeitgleich gezeigt werden.

Die Darstellungen wenigstens zweier Bildschirmfenster können sich insbesondere in Bezug auf die Anzahl der Dimensionen der Darstellungen unterscheiden.

Bei dem erfindungsgemäßen Verfahren können in einer Bildassistenzeinrichtung insbesondere in wenigstens einem Bildschirmfenster zeitgleich wenigstens zwei mehrdimensionale Darstellungen zumindest eines Abschnitts des Patientenkörpers dargestellt werden, wobei operationsrelevante Daten zusammengeführt und miteinander kombiniert werden können.

Diese operationsrelevanten Daten können 2D- und/oder 3D-Bild-Datensätze des Patienten und/oder weitere Daten, insbesondere zu Körpergröße, Gewicht, Alter des Patienten, vorangegangenen Eingriffen, sowie Daten aus vorherigen Untersuchungen umfassen und/oder solche Daten, die in unmittelbarem Zusammenhang mit einer Operation erstellt wurden.

Ein Aspekt der Erfindung besteht darin, dass auf Grund des erfindungsgemäßen Verfahrens zeitgleich unterschiedliche 3D-Patientenbilder und/oder 2D-Patientenbilder in Bildschirmfenstern dargestellt werden können, insbesondere wenigstens eine Darstellung eines 2D-Patientenbildes und wenigstens eine Darstellung eines 3D-Patientenbildes in wenigstens zwei Bildschirmfenstern des Bildschirms.

Insbesondere kann die Übertragung des Koordinatenbezugs automatisch von einer 2D-Darstellung auf eine 3D-Darstellung und umgekehrt erfolgen. Dies erfolgt insbesondere unabhängig von dem Format des dargestellten Patientenbildes (insbesondere 3D- oder 2D-Patientenbild), von dem Blickwinkel, von dem die jeweilige Darstellung gezeigt wird, oder davon, welchen axialen, sagittalen, koronalen oder sonstigen Schnitt des Patientenbildes die Arbeitsdarstellung und/oder weitere Bildschirmfenster zeigen.

Möglich ist es insbesondere, in einem Bildschirmfenster ein 3D-Patientenbild und im anderen Bildschirmfenster eine 2D-Schicht dieses 3D-Patientenbildes zu zeigen, wobei die 2D-Schicht insbesondere einen axialen Schnitt etwa durch die Wirbelsäule oder den Wirbel darstellt.

Es können beispielsweise neben einem 3D-Patientenbild drei 2D-Schichten dieses 3D-Patientenbildes dargestellt werden, von denen eine 2D-Schicht einen axialen Schnitt, die andere 2D-Schicht einen sagittalen und die dritte 2D-Schicht einen koronalen Schnitt durch den jeweiligen Bereich der Wirbelsäule zeigt.

Ferner kann beispielsweise in einem Bildschirmfenster eine Skizze, z.B. eine Wirbelsäulenskizze dargestellt werden. Während der Darstellung wird wenigstens ein weiteres Bildschirmfenster vergrößert und umfassender detailliert gezeigt. Ferner kann ein entsprechender Ausschnitt eines von dem Planer/Operateur etwa mit einem Cursor markierten Bereichs dieser Skizze an einem Patientenbild dargestellt werden. Zusätzlich kann das Patientenbild selbst dargestellt werden.

Der markierte Bereich kann insbesondere einen oder mehrere Wirbel der Wirbelsäule mit und ohne Bandscheiben umfassen, wobei in der Skizze die markierten Elemente, beispielsweise Wirbel und/oder Bandscheiben, farblich abgesetzt dargestellt werden können.

In der Arbeitsdarstellung können operationsrelevante Abschnitte eines Patientenkörpers aus unterschiedlichen mehrdimensionalen Patientenbildern zur Durchführung der präoperativen Planung dargestellt, vermessen und/oder bearbeitet werden.

Die Darstellung und/oder Bearbeitung von Patientenbildern also die Auswahl einer Arbeitsdarstellung, in den einzelnen Darstellungen kann mit Hilfe eines Cursors erfolgen.

Bestimmte Bereiche von Patientenbildern, wie beispielsweise Knochen, Gelenke, Weichteile und/oder Gewebe können insbesondere rechnergesteuert, beispielsweise mit Hilfe eines Cursors oder mit anderen Zeigevorrichtungen, mit Hilfe der wenigstens einen Arbeitsdarstellung der Bildassistenzeinrichtung in Korrelation mit einer Skizze dargestellt, vermessen und/oder bearbeitet werden. Insbesondere können einzelne Wirbel ausgewählt und markiert werden. Der ausgewählte Wirbel kann gegenüber den anderen Wirbeln farblich hervorgehoben werden.

Das endgültige Planungsergebnis, also die Darstellung des geplanten post-operativen Zustandes des Patienten, kann insbesondere in einer Navigationsansicht der Darstellung der Bildassistenzeinrichtung dargestellt werden. Dort werden anstelle der Implantat-Modelle Führungslinien angezeigt, welche die Ausrichtung und Positionierung des Implantats darstellen.

### Visualisierung auf Basis einer "virtual Reality" und/oder einer "mixed Reality" und/oder einer "augmented Reality"

Erfindungsgemäß erfolgt mit Hilfe der Bildassistenzeinrichtung und auf Grundlage einer erweiterten Realität eine Visualisierung der Planung der Maßnahmen.

Gleichfalls erfolgt die Visualisierung der Festlegung der Arbeitsschritte für die Operation am menschlichen Körper. Die Visualisierung der Ausbildung des Implantats ist erfindungsgemäß ebenfalls vorgesehen.

Die mit Hilfe der Bildassistenzeinrichtung und/oder auf Grundlage der erweiterten Realität erfolgten Visualisierung ist wenigstens eine Darstellung, die eine augmented Reality und/oder eine mixed Reality und/oder eine virtual Reality umfasst.

Die Visualisierung der Planung der Maßnahmen und/oder der Festlegung der Arbeitsschritte erfolgt auf Grundlage des Visierungsmittels, das auf Basis der erweiterten Realität funktioniert.

Die Festlegung der Arbeitsschritte sowie die Ausbildung des Implantats und/oder die jeweilige Anpassung und Modifikation erfolgt mittels eines Visualisierungsmittels, welches auf Grundlage der erweiterten Realität funktioniert.

Die Visualisierung funktioniert erfindungsgemäß auf Basis der virtual Reality und/oder der mixed Reality und/oder der augmented Reality.

Unter dem Begriff "virtual Reality" versteht die Erfindung die Darstellung und gleichzeitig die Wahrnehmung z.B. eines Menschen in einer computergenerierten, interaktiven virtuellen Umgebung, die in Echtzeit generiert wird.

Der Begriff "mixed Reality" bezeichnet die Wahrnehmung eines Menschen von seiner natürlichen Umgebung. In die Wahrnehmung der natürlichen Umgebung des Menschen werden vorzugsweise computererzeugte, virtuelle Realitäten eingeblendet.

Die Erfindung bezeichnet unter dem Begriff "augmented Reality" eine erweiterte Realität, die durch eine computergestützte Erweiterung der Wahrnehmung des Nutzers erreicht wird.

Mit anderen Worten bezeichnet die augmented Reality vorzugsweise eine visuelle Darstellung von Informationen, wodurch Bilder oder auch Videos mit Computer generierten Zusatzinformationen kombiniert werden.

Im Weiteren wird davon ausgegangen, dass das Visualisierungsmittel auf Basis einer augmented Reality funktioniert.

Die Erfindung sieht vor, dass die Daten, Werte und Informationen in dem Visualisierungsmittel in Form einer augmented Reality dargestellt werden.

Durch die Einblendung von Daten, Werten und Informationen in das Visualisierungsmittel in Form einer augmented Reality nimmt der Träger des Visualisierungsmittels die von ihm aufgerufenen Daten, Werte und Informationen als "vor sich im Raum schwebende Projektionen" wahr.

Der Träger des Visualisierungsmittels kann aufgrund der augmented Reality die Informationen, die "vor ihm im Raum schweben" verschieben, erweitern und/oder verändern.

Mit Hilfe der Augmneted Reality kann der Träger des Visualisierungsmittels, vorzugsweise, aber nicht ausschließlich, in einem "vor sich schwebenden Buch", das mit Hilfe der augmented Reality in sein Visualisierungsmittel eingeblendet wird, z.B. Seiten umblättern.

Er kann Passagen und Darstellungen des virtualen Buches extrahieren und gesondert im Speichermedium des Visualisierungsmittels abspeichern.

Mit Hilfe der augmented Reality kann sich der Träger des Visualisierungsmittels Daten, Werte und Informationen in sein Visualisierungsmittel einblenden lassen. Die eingeblendeten Daten, Werte und Informationen stehen der Person, die die Visite durchführt, als Zusatzinformation und/oder als Vergleichswerte bei der Behandlung des Patienten und/oder zur Beurteilung und/oder zur Beurteilung des Krankheitsverlaufs des Patienten zur Verfügung.

Zur Erstellung der Dokumentation über die Visite lässt sich der behandelnde Arzt den von ihm diktierten Bericht in Form einer augmented Reality-Darstellung in sein Visualisierungsmittel einblenden.

Der behandelnde Arzt sieht dabei, das von ihm erstellte Diktat in Form eines Textes, der "vor seinem Visualisierungsmittel im Raum schwebt".

Der behandelnde Arzt kann Fotos und Abbildungen, die er mit Hilfe des Visualisierungsmittels erstellt hat, in seine Dokumentation einfügen.

Die Funktion des Visualisierungsmittels auf Basis der augmented Reality ermöglicht, dass der behandelnde Arzt die Dokumentation der von ihm durchgeführten Visite am Krankenbett des Patienten erledigen kann.

Dadurch, dass das Visualisierungsmittel auf Basis der augmented Reality funktioniert, sieht der behandelnde Arzt sein Diktat in Textform umgesetzt, "vor sich im Raum schwebend".

Er ist dadurch in der Lage, den Text in zeitlichem Zusammenhang mit der Visite, oder noch während der Visite auf Korrektheit und Vollständigkeit zu überprüfen, um die Dokumentation der von ihm durchgeführten Visite z.B. an die anderen Teilnehmer seines Visitenteams und/oder vorzugsweise an das Informationssystem des Krankenhauses weiterzuleiten.

### Vorteile der Erfindung

Gegenüber dem Stand der Technik erweist sich die Erfindung aus vielen Gründen als vorteilhaft.

Bei der Planung eines operativen Eingriffs in eine kindliche Hüfte (Coxometrie) können beispielhaft, aber nicht ausschließlich, dem operativen Eingriff klinisch relevante Bemaßungen zur Beurteilung von Hüftgelenken zu Grunde gelegt werden. Zur Berücksichtigung national unterschiedlicher Kriterien bei der Planung eines operativen Eingriffs an der kindlichen Hüfte basiert das Verfahren auf Graduierungstabellen, die diese national verschiedenen Kriterien berücksichtigen, so dass das Verfahren international eingesetzt werden kann.

Ferner kann das erfindungsgemäße Verfahren auch zur Knie-Endoprothetik eingesetzt werden. Insbesondere können Achsfehlstellungen einer Knieprothese noch während der präoperativen Planung ermittelt und korrigiert werden.

Mit Hilfe des Verfahrens kann eine Korrektur der Fehlstellung beispielsweise des Beins abhängig von der jeweiligen Zielsetzung automatisch und/oder manuell vorgenommen werden. Hierzu werden die postoperativ zu erwartenden mechanischen Achsen, die Traglinie sowie alle relevanten Winkel ermittelt.

Erfindungsgemäß kann das Verfahren zudem zur Planung einer Osteotomie verwendet werden.

Hierzu kann die gezielte Durchtrennung einzelner oder mehrerer Knochen, beispielsweise um Fehlstellungen der Beinachsen oder der Hüftfehlstellung zu korrigieren, geplant werden. Das erfindungsgemäße Verfahren ermöglicht es weiter, eine oder mehrere Osteotomien nach der Art, der Anzahl, der Größe und der Lokalisierung festzulegen. Insbesondere femorale oder tibiale Umstellungs-Osteotomien können mit Hilfe des Verfahrens ebenso geplant werden, wie einfache oder mehrfache Osteotomien nach dem Open-Wedge-Verfahren oder nach dem Closed-Wedge-Verfahren.

Nach einer durchgeführten Korrektur können alle gelenkbezogenen, mechanischen Belastungsachsen und Gelenktangenten erfindungsgemäß einem Soll/Ist-Vergleich der Patientendaten mit Soll-, Ideal- und Normwerten oder Skizzen der menschlichen oder tierischen Anatomie unterzogen werden, um ein möglichst optimales post-operatives Ergebnis für den Patienten zu erzielen.

## Patentansprüche

1. Verfahren, bei dem mittels eines Computers Maßnahmen zur Durchführung einer Operation geplant und/oder Arbeitsschritte für die Operation an einem menschlichen oder einem tierischen Körper festgelegt werden,
- wobei die Operation den Einsatz wenigstens eines Implantats umfasst,
- wobei der Computer ein Speicherwerk umfasst, in das Daten und/oder Darstellungen in Form mehrdimensionaler Bilder eingespeichert werden, wobei
- die Daten und/oder Darstellungen den Körper eines Patienten betreffen, der der Operation unterzogen wird, und/oder
- die Daten und/oder Darstellungen eine Ausbildung des Implantats betreffen, das bei der Operation zum Einsatz kommt, wobei
- der Computer eine Bildassistenzeinrichtung umfasst, in die aus dem Speicherwerk, ausgewählte Daten und/oder Darstellungen eingespeist werden,
- wobei im Computer eine Simulation der geplanten Maßnahmen und/oder der Arbeitsschritte für die Operation durchgeführt und ausgewertet wird, die die Darstellung von Wechselwirkungen des Implantats mit dem Körper des Patienten umfasst, und
- die geplanten Maßnahmen und/oder die Arbeitsschritte für die Operation und/oder die Ausbildung des Implantats in Abhängigkeit des Auswertungsergebnisses der Simulation angepasst und/oder modifiziert werden,
- wobei mit Hilfe der Bildassistenzeinrichtung und auf Grundlage einer erweiterten Realität eine Visualisierung der Planung der Maßnahmen und/oder der Festlegung der Arbeitsschritte für die Operation und/oder eine Visualisierung der Ausbildung des Implantats erfolgt.

2. Verfahren nach Anspruch 1, bei dem das Speicherwerk ein PACS-System (Bildarchivierungs- und Kommunikationssystem) eines Krankenhauses umfasst.

3. Verfahren nach Anspruch 1, **dadurch gekennzeichnet, dass** die in die Bildassistenzeinrichtung eingespeisten Daten und/oder Darstellungen be- und/oder verarbeitet werden.

4. Verfahren nach Anspruch 1, **dadurch gekennzeichnet, dass** die in die Bildassistenzeinrichtung eingespeisten Daten und/oder Darstellungen, Implantat-Typen, Abmessungen von Implantaten und/oder Implantat-Varianten umfassen.

5. Verfahren nach Anspruch 1, **dadurch gekennzeichnet, dass** im Rahmen eines Soll-Ist-Vergleichs die vor Beginn der Operation geplanten Maßnahmen mit den tatsächlich vor Beginn der Operation durchgeführten Maßnahmen und/oder die geplanten Arbeitsschritte mit den tatsächlich während der Operation durchgeführten Arbeitsschritten verglichen werden.

6. Verfahren nach Anspruch 1, **dadurch gekennzeichnet, dass** die mit Hilfe der Bildassistenzeinrichtung und auf Grundlage der erweiterten Realität erfolgten Visualisierung Darstellungen sind, die eine "augmented Reality" und/oder eine "mixed Reality" und/oder eine "virtual Reality" umfassen.

7. Verfahren nach Anspruch 1, **dadurch gekennzeichnet, dass** das mehrdimensionale Bild wenigstens eine 2D-Darstellung oder eine 3D-Darstellung ist. Mehr als 3D-Darstellung möglich?

8. Verfahren nach Anspruch 1, **dadurch gekennzeichnet, dass** die Simulation und/oder Anpassung oder Modifizierung und/oder die Visualisierung durch die Bildassistenzeinrichtung ausgeführt wird.

9. Verfahren nach Anspruch 1, **dadurch gekennzeichnet, dass** Informationen zu den angepassten oder modifizierten Maßnahmen und/oder zu den angepassten oder modifizierten Arbeitsschritten und/oder die Daten und/oder die Darstellung des angepassten oder modifizierten Implantats zur Abspeicherung in das Speicherwerk übermittelt werden.

10. Verfahren nach Anspruch 1, **dadurch gekennzeichnet, dass** die Visualisierung der Planung der Maßnahmen und/oder der Festlegung der Arbeitsschritte und/oder der Ausbildung des Implantats und/oder der jeweiligen Anpassung und Modifikation mittels eines, auf Grundlage der erweiterten Realität funktionierenden Visualisierungsmittels erfolgt.

11. Verfahren nach Anspruch 1, **dadurch gekennzeichnet, dass** das Visualisierungsmittel mit Hilfe einer Gestensteuerung und/oder einer Sprachsteuerung gesteuert wird.

12. System zur Durchführung des Verfahrens nach einem der vorausgehenden Ansprüche, bei dem mittels eines Computers Maßnahmen zur Durchführung einer Operation geplant und/oder Arbeitsschritte für die Operation an einem menschlichen oder einem tierischen Körper festlegbar sind,
- wobei die Operation den Einsatz wenigstens eines Implantats umfasst,
- wobei der Computer ein Speicherwerk umfasst, in das Daten und/oder Darstellungen in Form mehrdimensionaler Bilder einspeicherbar sind, wobei
- die Daten und/oder Darstellungen den Körper eines Patienten betreffen, der der Operation unterzogen wird, und/oder
- die Daten und/oder Darstellungen eine Ausbildung des Implantats betreffen, das bei der Operation einsetzbar ist, wobei
- der Computer eine Bildassistenzeinrichtung umfasst, in die aus dem Speicherwerk, ausgewählte Daten und/oder Darstellungen einspeicherbar sind,
- wobei im Computer eine Simulation der geplanten Maßnahmen und/oder der Arbeitsschritte für die Operation durchgeführt und ausgewertet wird, die die Darstellung von Wechselwirkungen des Implantats mit dem Körper des Patienten umfasst, und
- die geplanten Maßnahmen und/oder die Arbeitsschritte für die Operation und/oder die Ausbildung des Implantats in Abhängigkeit des Auswertungsergebnisses der Simulation anpassbar und modifizierbar sind,
- wobei mit Hilfe der Bildassistenzeinrichtung und auf Grundlage einer erweiterten Realität eine Visualisierung der Planung der Maßnahmen und/oder der Festlegung der Arbeitsschritte für die Operation und/oder eine Visualisierung der Ausbildung des Implantats ausführbar ist.
